# FASCICULE DE BREVET EUROPEEN

(11) **EP 2 315 553 B1**
(45) Date de publication et mention de la délivrance du brevet: **25.03.2015**
(21) Numéro de dépôt: 09784374.2
(22) Date de dépôt: 08.04.2009
(51) Int. Cl.: A61C 5/04, A61C 9/00

(54) **DISPOSITIF POUR INJECTER DANS UN LOGEMENT CANALAIRE UN MATERIAU DE REMPLISSAGE EN PHASE FLUIDE.**
VORRICHTUNG ZUR INJEKTION EINES FÜLLSTOFFES IN DER FLUIDPHASE IN EINEN KANALRAUM
DEVICE FOR INJECTING A FILLING MATERIAL IN THE FLUID PHASE INTO A CANAL SPACE.

(30) Priorité: 17.07.2008 FR 0854866; 22.09.2008 FR 0856343
(43) Date de publication de la demande: 04.05.2011
(73) Titulaire: Koubi, Stéphen, 13001 Marseille (FR)
(72) Inventeur: KOUBI, Stéphen, 13001 Marseille (FR)
(74) Mandataire: Roman, Alexis
(86) Numéro de dépôt international: PCT/FR2009/050624
(87) Numéro de publication internationale: WO 2010/007257

(56) Documents cités:
- EP-A- 0 035 481
- EP-A- 0 815 802
- EP-A- 1 790 308
- WO-A-82/03761
- US-A- 6 079 979
- US-B1- 6 422 865
- US-B1- 6 786 724

## Description

### Domaine technique de l'invention.

La présente invention a pour objet principal un dispositif pour injecter dans un canal dentaire un matériau de remplissage en phase fluide, et notamment en phase visqueuse.

Elle concerne le domaine technique de l'odontologie et de la dentisterie restauratrice et plus particulièrement celui des embouts d'injection intra-orale permettant d'injecter un matériau de remplissage, de scellement ou d'empreinte dans un logement canalaire.

### État de la technique.

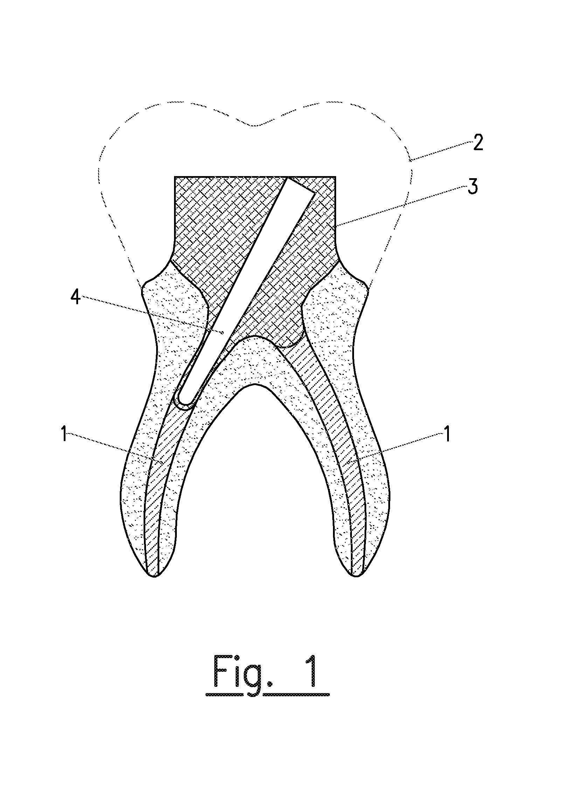

En dentisterie classique, il est fréquent d'avoir à dévitaliser une dent. En se rapportant à la figure 1, après cette opération, les canaux radiculaires 1 doivent être comblés ou obturés par une pâte afin de les protéger d'éventuelles intrusions bactériennes. Cette pâte est ensuite destinée à durcir suite à une réaction chimique et/ou thermique.

Il est également fréquent d'utiliser des couronnes prothétiques pour réparer des dents endommagées. En se référant à la figure 1, pour que la couronne 2 (représentée en pointillés) tienne, il est nécessaire que le pilier prothétique 3 soit suffisamment important. C'est pourquoi, quand il y a eu une fracture importante de la dent, on est obligé de reconstituer artificiellement ce pilier 3, à l'aide de matériaux composites ou métalliques. Si le manque de matière est conséquent, il est possible de s'encrer dans les canaux radiculaires 1 pour avoir plus de résistance : c'est ce que l'on appelle une reconstitution corono-radiculaire. Dans ce cas, on peut utiliser des tenons 4 fibrés ou non, qui sont scellés dans des logements canalaires réalisés dans les canaux radiculaires préalablement obturés. En général, le scellement des tenons est réalisé par l'intermédiaire de résines ou de colles aptes à durcir par réaction chimique. Ce tenon 4 est ensuite noyé dans un matériau de reconstitution, afin de reconstituer le pilier prothétique 3 sur lequel sera fixé la couronne 2.

Il est également possible d'utiliser des tenons radiculaires réalisés en une seule pièce et sur mesure (ou inlay-core en anglais). Ils remplissent principalement deux rôles : encrage dans le canal radiculaire et reconstitution en une seule pièce du pilier prothétique 3. Cette solution se réalise en deux temps : la prise préalable d'une empreinte au cours de laquelle du silicone est injecté dans le logement canalaire. Grâce à cette empreinte, le prothésiste aura la possibilité de confectionner l'inlay-core qui s'adaptera parfaitement à l'anatomie canalaire. La deuxième étape est le scellement de cette pièce prothétique dans le logement canalaire.

Les matériaux de scellement ou d'empreinte introduits dans les logements canalaires sont dénommés par la suite « matériaux de remplissage en phase fluide ». Ils sont généralement obtenus à partir du mélange d'une base et d'un catalyseur.

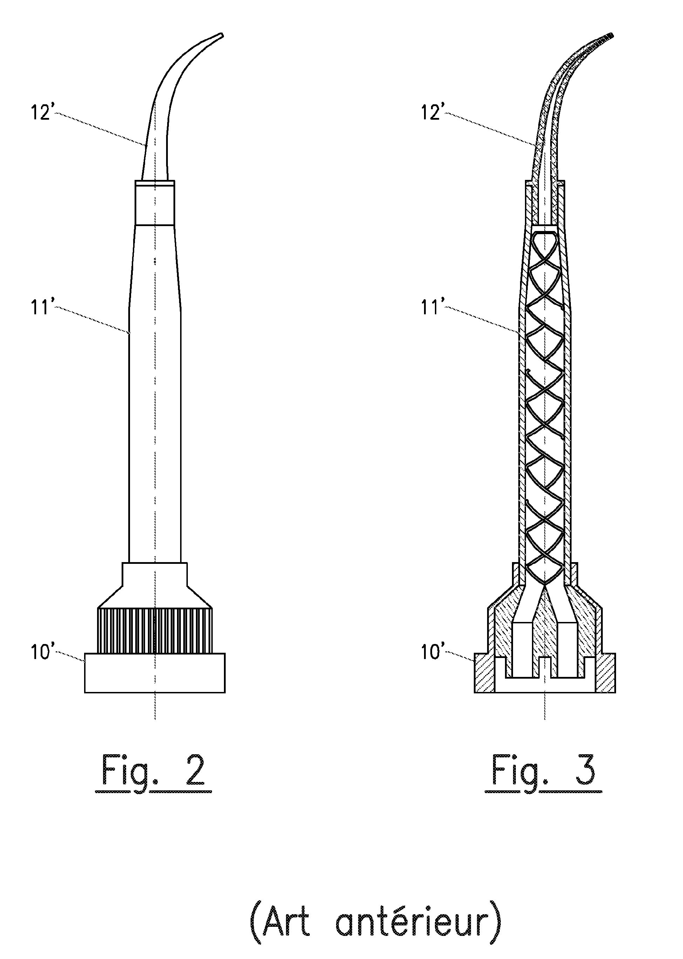

A ce jour, on connaît des dispositifs permettant d'injecter ces matériaux de remplissage en phase fluide. Ces dispositifs connus de l'art antérieur sont par exemple représentés sur les figures 2 et 3. Ils comportent généralement :
- un embout d'adaptation 10',
- un auto-mélangeur 11' relié audit embout d'adaptation,
- un embout d'injection intra-oral 12' disposé à l'extrémité dudit auto-mélangeur.

L'embout d'adaptation 10' est configuré pour se fixer sur l'orifice de sortie d'une cartouche présentant généralement des compartiments étanches contenant la base et le catalyseur constitutifs du matériau de remplissage. Généralement, la cartouche se présente sous la forme d'une seringue ou d'un pistolet équipé d'un moyen pour faire sortir la base et le catalyseur vers un orifice de sortie sur lequel est connecté l'embout d'adaptation 10'. Le mélange de la base et du catalyseur amorce la réaction chimique qui permettra de faire durcir le matériau final une fois injecté dans le logement canalaire. Le mélange est réalisé au moyen de l'auto-mélangeur 11' qui consiste en une hélice multiple configurée pour mélanger automatiquement, et de manière très homogène, la base et le catalyseur. Ce type de dispositif est bien connu de l'homme du métier et sont par exemple commercialisés par la société MIXPACK® sous l'appellation « EMBOUTS MÉLANGEURS » et « EMBOUTS INTRA-ORAUX ».

Le document brevet EP 0.815.802 (3M) décrit un dispositif de ce type dans lequel l'embout d'injection est réalisé dans un matériau à mémoire de forme de manière à pouvoir le plier selon une orientation voulue par une simple pression des doigts. Une extrémité évasée conique permet de maintenir en position l'embout d'injection dans l'auto-mélangeur tout en lui laissant un degré de liberté en rotation autour de son axe longitudinal. Cette configuration apparaît être particulièrement contraignante lors de la conception du dispositif. En effet, il est nécessaire de positionner préalablement l'embout d'injection dans le tube plastique formant l'auto-mélangeur avant de mettre en place la double hélice. Cette dernière permet de bloquer en position l'embout d'injection à l'extrémité de l'auto-mélangeur.

Bien que largement utilisés par les praticiens, ces dispositifs connus de l'art antérieur, y compris celui décrit dans le document brevet EP 0.815.802, présentent certains inconvénients. En effet, l'embout d'injection intra-oral 12' se présente généralement sous la forme d'un tube conique d'environ 20 mm de long. Son diamètre externe à l'extrémité distale est d'environ 1.5 mm et le diamètre externe à l'extrémité proximale est d'environ 3 mm. Or, en pratique, les logements canalaires ont un diamètre d'environ 1.5 mm, sur une profondeur de 8 mm à 15 mm. L'extrémité distale de l'embout intra-oral 12' n'a donc pas la possibilité d'atteindre la partie la plus apicale du logement canalaire, le matériau de remplissage en phase fluide ne pouvant pénétrer dans tout l'espace canalaire préparé. De plus, l'embout d'injection 12' est généralement réalisé en plastique, de sorte qu'il n'est pas possible de modifier son orientation initiale. Ceci est particulièrement contraignant, car avec cette configuration imposée, le praticien peut avoir de grandes difficultés à atteindre le logement canalaire selon la position de la dent dans laquelle il est réalisé.

Le document brevet EP 0.035.481 (SÖDERSTRÖM) divulgue un dispositif permettant la production d'un noyau pour une dent à racine préparée. Une matière de prise d'empreinte est injectée au fond du logement canalaire pour le remplir successivement du fond vers l'extérieur tout en y expulsant l'air. L'injection est effectuée à l'aide d'une aiguille à injection munie d'un corps cylindrique et d'un piston. Sauf à la modifier largement, cette aiguille d'injection très spécifique ne peut en aucun cas remplacer l'embout d'injection d'un dispositif d'injection avec auto-mélangeur du type décrit dans le document brevet EP 0.815.802.

Face à cet état des choses, le principal objectif de l'invention est de perfectionner les dispositifs d'injection avec auto-mélangeur du type décrit dans le document brevet EP 0.815.802, en vue d'injecter avec une grande rigueur, un matériau de remplissage en phase fluide dans un logement canalaire, de manière à obtenir un remplissage homogène pratiquement exempt de bulles d'air.

Un autre objectif de l'invention est de proposer un dispositif d'injection permettant de facilement accéder à la partie la plus apicale du logement canalaire.

Encore un autre objectif de l'invention est de proposer un dispositif d'injection de conception simple et dont l'utilisation est particulièrement aisée pour le praticien.

### Divulgation de l'invention.

La solution proposée par l'invention est un dispositif d'injection du type décrit dans le document brevet EP 0.815.802, remarquable en ce que :
1. l'embout d'injection est un tube dont l'extrémité distale a un diamètre externe inférieur ou égal à 1.5 mm sur une longueur supérieure ou égale à 8 mm,
2. l'embout d'injection est réalisé dans un matériau à mémoire de forme de manière à pouvoir le plier selon une orientation voulue,
3. l'embout d'injection comporte sur sa surface extérieure :
   - soit des nervures circulaires définissant une gorge, l'extrémité supérieure de l'auto-mélangeur étant moulée dans cette gorge de manière à ce que ledit embout d'injection soit bloqué en position dans ladite extrémité supérieure tout en gardant un degré de liberté en rotation autour de son axe de symétrie,
   - soit une ou plusieurs nervures circulaires parallèles entre elles, l'extrémité supérieure de l'auto-mélangeur étant moulée sur cette nervure circulaire de manière à ce que ledit embout d'injection soit bloqué en position dans ladite extrémité supérieure tout en gardant un degré de liberté en rotation autour de son axe de symétrie.

La première caractéristique permet de remplir intégralement le logement canalaire, l'extrémité distale de l'embout d'injection pouvant aisément atteindre la partie la plus apicale dudit logement. La seconde caractéristique offre au praticien la possibilité de conformer l'embout d'injection selon la position de la dent à traiter et l'orientation du logement canalaire à remplir. Le praticien a par exemple la possibilité de choisir l'endroit où l'embout d'injection sera plié ainsi que son rayon de courbure pour s'adapter au mieux à la configuration du logement canalaire. La troisième caractéristique permet de simplifier la conception du dispositif d'injection en obtenant une pièce monobloc obtenue directement par moulage et offre au praticien la possibilité de faire pivoter seulement l'embout d'injection, et non l'ensemble du dispositif, de manière à orienter correctement ledit embout dans la direction de la dent à traiter. Ces caractéristiques se combinent pour atteindre les objectifs précités.

De manière avantageuse, l'embout d'injection est un tube cylindrique dont l'extrémité distale a un diamètre externe compris entre 0,75 mm et 0,95 mm, préférentiellement 0,8 mm. Cette plage de diamètres permet non seulement d'injecter un grand nombre de matériaux de remplissage, de scellement ou d'empreinte en phase visqueuse, mais encore d'insérer aisément le conduit d'injection dans tout type de logement canalaire.

Selon une caractéristique préférée de réalisation, l'embout d'injection est en métal, de sorte que le praticien puisse facilement le conformer pour lui donner la forme voulue.

De manière préférée, l'embout d'injection a une longueur supérieure ou égale à 15 mm.

Un autre aspect de l'invention est un système pour injecter dans un logement canalaire un matériau de remplissage en phase fluide, comportant :
- une cartouche contenant dans des compartiments étanches une base et un catalyseur à mélanger pour obtenir ledit matériau de remplissage en phase fluide, ladite cartouche étant équipée d'un moyen pour faire sortir ladite base et ledit catalyseur vers un orifice de sortie,
- un auto-mélangeur disposé au niveau de l'orifice de sortie de la cartouche et configuré pour mélanger les matériaux primaires,
- un embout d'injection intra-oral conforme aux caractéristiques précédentes et agencé à l'extrémité supérieure de l'auto-mélangeur.

Encore un autre aspect de l'invention concerne un nécessaire dentaire prêt à être utilisé comprenant :
- une cartouche contenant dans des compartiments étanches des matériaux fluides primaires à mélanger, ladite cartouche étant équipée d'un moyen pour faire sortir lesdits matériaux primaires vers un orifice de sortie,
- le dispositif d'injection conforme aux caractéristiques précédentes.

### Description des figures.

D'autres avantages et caractéristiques de l'invention apparaîtront mieux à la lecture de la description d'un mode de réalisation préféré qui va suivre, en référence aux dessins annexés, réalisés à titre d'exemples indicatifs et non limitatifs et sur lesquels :
- la figure 1 précitée est une vue schématique en coupe d'une molaire montrant l'anatomie canalaire et la mise en place des différents éléments prothétiques,
- les figures 2 et 3 précitées représentent schématiquement un dispositif d'injection connu de l'art antérieur,
- la figure 4 représente schématiquement un dispositif d'injection conforme à l'invention,
- les figures 5a et 5b sont des vues schématiques en coupe verticale du dispositif d'injection conforme à l'invention,
- la figure 6a représente schématiquement un dispositif d'injection conforme à l'invention monté sur une cartouche intégrée dans une seringue.
- la figure 6b représente schématiquement un dispositif d'injection conforme à l'invention monté sur une cartouche destinée à être montée sur un pistolet.

### Modes de réalisation de l'invention.

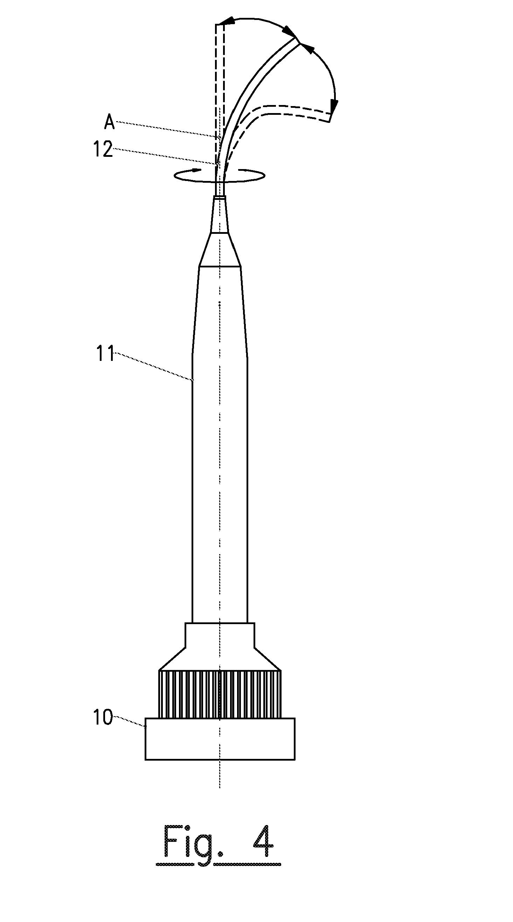

En se rapportant aux figures 4 à 6b, le dispositif d'injection objet de l'invention comporte :
- un embout d'adaptation 10 configuré pour se fixer sur l'orifice de sortie d'une cartouche contenant directement le matériau de remplissage en phase fluide ou une base et un catalyseur à mélanger pour obtenir ledit matériau,
- un auto-mélangeur 11 relié audit embout d'adaptation,
- un embout d'injection intra-oral 12 disposé à l'extrémité supérieure dudit auto-mélangeur.

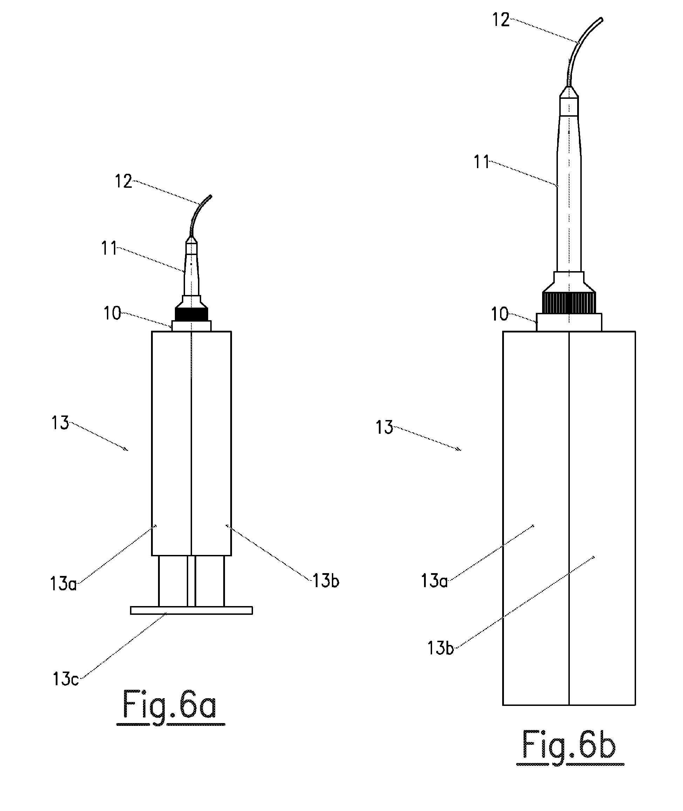

En pratique, l'embout d'adaptation 10 est un embout cylindrique en plastique rigide, de diamètre compris entre 10 mm et 25 mm, venant se viser, se cliper ou pouvant être directement moulé sur l'orifice de sortie de la cartouche 13. En se rapportant aux figures 6a et 6b, la cartouche 13 comporte généralement deux compartiments 13a, 13b contenant respectivement une base et un catalyseur, qui une fois mélangés permettront d'obtenir le matériau de remplissage en phase fluide. La cartouche 13 est équipée d'un moyen 13c, typiquement le piston d'une seringue (figure 6a) ou un pistolet, permettant de faire sortir, à la demande du praticien, la base et le catalyseur vers un orifice de sortie sur lequel est disposé l'embout d'adaptation 10. Ces cartouches 13 sont celles habituellement utilisées par le praticien. En pratique, il s'agit de cartouches 13 intégrées dans une seringue (plus connues sous la dénomination SMART KIT®) (figure 6a) ou celles, plus volumineuses, destinées à être montées sur un pistolet (figure 6b). Lorsque le dispositif objet de l'invention est destiné être monté sur des cartouches intégrées dans des seringues, l'embout d'adaptation 10 aura un diamètre d'environ 10 mm et lorsqu'il sera monté sur des cartouches pour pistolets, ledit embout d'adaptation aura un diamètre d'environ 15 mm. Il est à noter que l'on pourrait prévoir d'utiliser une cartouche contenant directement le matériau de remplissage en phase fluide déjà préparé. Dans ce cas, un seul compartiment est nécessaire.

L'embout d'adaptation 10 intègre avantageusement deux tubes 10a, 10b qui, lorsque ledit embout est positionné sur la cartouche 13, pénètrent dans chacun des compartiments 13a, 13b. Les deux tubes 10a, 10b, se rejoignent à la base de l'auto-mélangeur 11.

L'auto-mélangeur 11 est fixé à l'extrémité supérieure de l'embout d'adaptation 10. Il se présente sous la forme d'un tube plastique ayant une longueur comprise entre environ 20 mm et environ 50 mm et un diamètre compris entre environ 4 mm et environ 6 mm, à l'intérieur duquel est agencée une double hélice 11a configurée pour mélanger de manière homogène la base et le catalyseur. Par exemple, lorsque le dispositif objet de l'invention sera monté sur des cartouches intégrées dans des seringues, l'auto-mélangeur 11 aura une longueur d'environ 20 mm et un diamètre d'environ 4 mm (figure 6a) et lorsqu'il sera monté sur des cartouches pour pistolets (figure 6b), ledit auto-mélangeur aura une longueur d'environ 50 mm et un diamètre d'environ 6 mm. L'embout d'adaptation 10 et l'auto-mélangeur 11 sont connus de l'homme du métier et sont par exemple commercialisés par la société MIXPACK® sous l'appellation « EMBOUTS MÉLANGEURS ».

Dans le cas où la cartouche 13 contient le matériau de remplissage directement préparé, l'auto-mélangeur 11 n'est plus indispensable et/ou peut uniquement servir à travailler ledit matériau avant son injection pour amorcer la réaction chimique. De même, si l'auto-mélangeur 11 est directement moulé au niveau de l'orifice de sortie de la cartouche 13, l'embout d'adaptation 10 n'est plus utile.

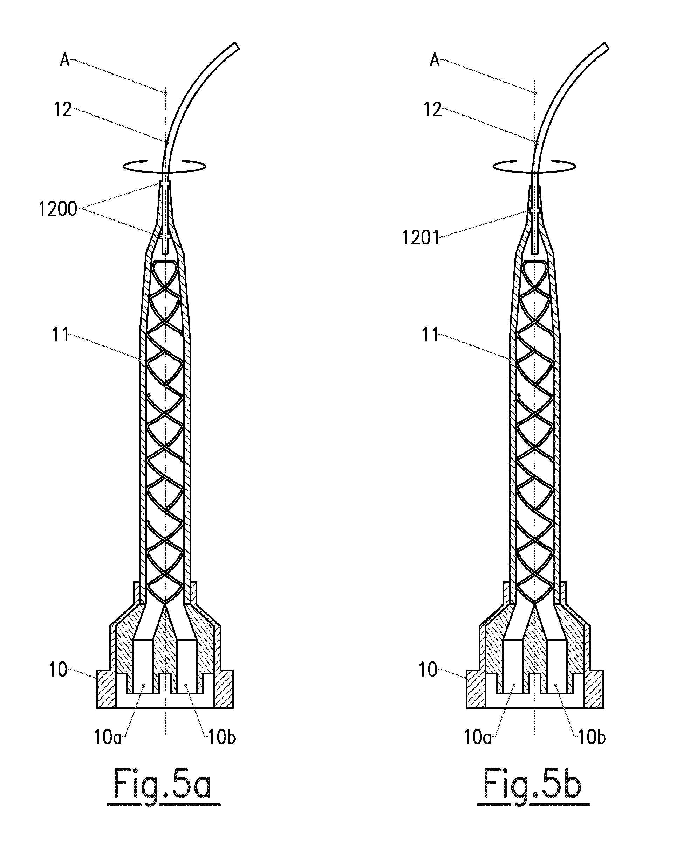

L'embout d'injection intra-oral 12 est disposé à l'extrémité supérieure de l'auto-mélangeur 11 où la base et le catalyseur arrivent intimement mélangés. En se rapportant aux figures 4, 5a et 5b, l'embout d'injection 12 se présente sous la forme d'un tube dont l'extrémité distale a un diamètre externe inférieur ou égal à 1.5 mm, sur une longueur supérieure ou égale à 8 mm. En pratique, l'embout d'injection 12 est un tube cylindrique ayant un diamètre externe constant inférieur ou égal à 1.5 mm, avantageusement entre 0,75 mm et 0,95 mm, préférentiellement de 0,8 mm, et de longueur supérieure ou égale à 15 mm, préférentiellement égale à 18 mm. Toutefois, une longueur de 30 mm voire davantage peut être prévue, le but étant d'avoir un embout d'injection 12 suffisamment long pour que son extrémité distale puisse atteindre le fond du logement canalaire, quelle que soit la position de la dent à traiter. Dans une variante de réalisation, on peut envisager d'employer un tube sensiblement conique d'environ 15 mm de long, et dont le diamètre externe à l'extrémité distale est d'environ 0,8 mm et le diamètre externe à l'extrémité proximale est d'environ 1.5 mm. Le diamètre interne de l'embout d'injection 12 est d'environ 0.8 mm.

Conformément à l'invention, l'embout d'injection 12 est réalisé dans un matériau à mémoire de forme de manière à pouvoir le plier selon une orientation voulue. On préfère utiliser un embout 12 en métal mais d'autres matériaux équivalent tels que des plastiques thermo-formables peuvent être envisagés. L'avantage d'un embout d'injection 12 en métal, est qu'il peut facilement et rapidement être conformé à la main (ou à l'aide d'une pince) par le praticien afin de lui donner une géométrie voulue, adaptée à la position de la dent à traiter et à la configuration du logement canalaire à remplir. Dans le cas de plastiques thermo-formable, le praticien devra préalablement chauffer l'embout d'injection 12 afin de lui donner la forme voulue.

Les traits en pointillés sur la figure 4 représentent schématiquement différentes configurations possibles de l'embout d'injection 12. Par exemple, pour le traitement d'une incisive ou d'une canine, le praticien préférera disposer d'un embout d'injection 12 sensiblement droit tandis que le traitement d'une molaire nécessitera de plier ledit embout à un endroit plus ou moins proche de l'extrémité distale.

En se rapportant à la figure 5a, l'embout d'injection 12 comporte sur sa surface extérieure des nervures circulaires 1200 définissant une gorge. L'extrémité supérieure de l'auto-mélangeur 11 est ensuite moulée dans cette gorge ainsi formée. Dans cette configuration, l'embout d'injection 12 est bloqué en position dans l'extrémité supérieure de l'auto-mélangeur 11 tout en gardant un degré de liberté en rotation autour de son axe de symétrie A.

Dans une variante de réalisation représentée sur la figure 5b, l'embout d'injection comportera sur sa surface extérieure une nervure circulaire 1201, l'extrémité supérieure de l'auto-mélangeur 11 étant ensuite moulée sur cette nervure circulaire. Dans cette configuration, l'embout d'injection 12 est ici encore bloqué en position dans l'extrémité supérieure de l'auto-mélangeur 11 tout en gardant un degré de liberté en rotation autour de son axe de symétrie A. Un résultat similaire serait obtenu en disposant sur la surface extérieure de l'embout d'injection 12, plusieurs nervures circulaires parallèles entre-elles.

Le dispositif d'injection objet de l'invention peut être un accessoire indépendant des cartouches ou au contraire être directement intégré à celles-ci lors de leur fabrication. Dans le cas où le dispositif d'injection est un simple accessoire, l'auto-mélangeur 11 doit être équipé de l'embout d'adaptation 10. Le dispositif d'injection fait alors avantageusement partie d'un nécessaire dentaire prêt à être utilisé, communément dénommé Kit, comprenant en outre une cartouche du type décrite précédemment.

## Revendications

1. Dispositif pour injecter dans un logement canalaire un matériau de remplissage en phase fluide, ledit dispositif comportant un embout d'adaptation (10), un auto-mélangeur (11) relié audit embout d'adaptation, un embout d'injection intra-oral (12) disposé à l'extrémité supérieure dudit auto-mélangeur,
**se caractérisant par le fait que** ledit embout d'injection est un tube dont l'extrémité distale a un diamètre externe inférieur ou égal à 1.5 mm sur une longueur supérieure ou égale à 8 mm,
**par le fait que** ledit embout d'injection est réalisé dans un matériau à mémoire de forme de manière à pouvoir le plier selon une orientation voulue,
et **par le fait que** ledit embout d'injection comporte sur sa surface extérieure :
• soit des nervures circulaires (1200) définissant une gorge, l'extrémité supérieure de l'auto-mélangeur (11) étant moulée dans cette gorge de manière à ce que ledit embout d'injection soit bloqué en position dans ladite extrémité supérieure tout en gardant un degré de liberté en rotation autour de son axe de symétrie (A),
• soit une ou plusieurs nervures circulaires (1201) parallèles entre elles, l'extrémité supérieure de l'auto-mélangeur (11) étant moulée sur cette nervure circulaire de manière à ce que ledit embout d'injection soit bloqué en position dans ladite extrémité supérieure tout en gardant un degré de liberté en rotation autour de son axe de symétrie (A).

2. Dispositif selon la revendication 1, dans lequel l'embout d'injection (12) est un tube cylindrique dont l'extrémité distale a un diamètre externe compris entre 0,75 mm et 0,95 mm.

3. Dispositif selon l'une des revendications précédentes, dans lequel l'embout d'injection (12) est un tube cylindrique dont l'extrémité distale a un diamètre externe de 0,8 mm.

4. Dispositif selon l'une des revendications précédentes, dans lequel l'embout d'injection (12) est en métal.

5. Dispositif selon l'une des revendications précédentes, dans lequel l'embout d'injection (12) a une longueur supérieure ou égale à 15 mm.

6. Système pour injecter dans un logement canalaire un matériau de remplissage en phase fluide, comportant :
• une cartouche (13) contenant dans des compartiments étanches (13a, 13b) une base et un catalyseur à mélanger pour obtenir ledit matériau de remplissage en phase fluide, ladite cartouche étant équipée d'un moyen (13c) pour faire sortir ladite base et ledit catalyseur vers un orifice de sortie,
• un auto-mélangeur (11) disposé au niveau de l'orifice de sortie de la cartouche (13) et configuré pour mélanger la base et le catalyseur,
• un embout d'injection (12) intra-oral disposé à l'extrémité supérieure de l'auto-mélangeur (11),
**se caractérisant par le fait que** ledit embout d'injection est un tube dont l'extrémité distale a un diamètre externe inférieur ou égal à 1.5 mm sur une longueur supérieure ou égale à 8 mm,
**par le fait que** ledit embout d'injection est réalisé dans un matériau à mémoire de forme de manière à pouvoir le plier selon une orientation voulue,
**et par le fait que** ledit embout d'injection comporte sur sa surface extérieure :
• soit des nervures circulaires (1200) définissant une gorge, l'extrémité supérieure de l'auto-mélangeur (11) étant moulée dans cette gorge de manière à ce que ledit embout d'injection soit bloqué en position dans ladite extrémité supérieure tout en gardant un degré de liberté en rotation autour de son axe de symétrie (A),
• soit une ou plusieurs nervures circulaires (1201) parallèles entre elles, l'extrémité supérieure de l'auto-mélangeur (11) étant moulée sur cette nervure circulaire de manière à ce que ledit embout d'injection soit bloqué en position dans ladite extrémité supérieure tout en gardant un degré de liberté en rotation autour de son axe de symétrie (A).

7. Système selon la revendication 6, dans lequel l'embout d'injection (12) est un tube cylindrique dont l'extrémité distale a un diamètre externe compris entre 0,75 mm et 0,95 mm.

8. Système selon l'une des revendications 6 ou 7, dans lequel l'embout d'injection (12) est un tube cylindrique dont l'extrémité distale a un diamètre externe de 0,8 mm.

9. Système selon l'une des revendications 6 à 8, dans lequel l'embout d'injection (12) est en métal.

10. Système selon l'une des revendications 6 à 9, dans lequel l'embout d'injection (12) a une longueur supérieure ou égale à 15 mm.

11. Nécessaire dentaire prêt à être utilisé comprenant :
• une cartouche (13) contenant dans des compartiments étanches (13a, 13b) une base et un catalyseur à mélanger pour obtenir un matériau de remplissage en phase fluide, ladite cartouche étant équipée d'un moyen (13c) pour faire sortir ladite base et ledit catalyseur vers un orifice de sortie,
• le dispositif d'injection conforme aux revendications 1 à 5.

## Patentansprüche

1. Vorrichtung zur Injektion eines Füllstoffs in Fluidphase in eine kanalartige Aufnahme, wobei die Vorrichtung ein Adapteransatzstück (10), einen mit dem Adapteransatzstück verbundenen automatischen Mischer (11) und ein am oberen Ende des automatischen Mischers angeordnetes Ansatzstück (12) zur intraoralen Injektion umfasst,
**dadurch gekennzeichnet, dass** das Injektionsansatzstück eine Röhre ist, deren distales Ende einen äußeren Durchmesser von kleiner oder gleich 1,5 mm bei einer Länge von größer oder gleich 8 mm hat, dass das Injektionsansatzstück aus einem Formgedächtnismaterial ausgeführt ist, so dass es in einer gewünschten Ausrichtung gebogen werden kann, und dass das Injektionsansatzstück an seiner Außenfläche Folgendes umfasst:
• entweder kreisförmige Rippen (1200), die eine Kehle definieren, wobei das obere Ende des automatischen Mischers (11) so in diese Kehle geformt ist, dass das Injektionsansatzstück in dem oberen Ende in Position arretiert ist, wobei es einen Drehfreiheitsgrad um seine Symmetrieachse (A) beibehält,
• oder eine oder mehrere kreisförmige, zueinander parallele Rippen (1201), wobei das obere Ende des automatischen Mischers (11) so auf diese kreisförmige Rippe geformt ist, dass das Injektionsansatzstück in dem oberen Ende in Position arretiert ist, wobei es einen Drehfreiheitsgrad um seine Symmetrieachse (A) beibehält.

2. Vorrichtung nach Anspruch 1, wobei das Injektionsansatzstück (12) eine zylindrische Röhre ist, deren distales Ende einen externen Durchmesser zwischen 0,75 mm und 0,95 mm hat.

3. Vorrichtung nach einem der vorhergehenden Ansprüche, wobei das Injektionsansatzstück (12) eine zylindrische Röhre ist, deren distales Ende einen externen Durchmesser von 0,8 mm hat.

4. Vorrichtung nach einem der vorhergehenden Ansprüche, wobei das Injektionsansatzstück (12) aus Metall ist.

5. Vorrichtung nach einem der vorhergehenden Ansprüche, wobei das Injektionsansatzstück (12) eine Länge von größer oder gleich 15 mm hat.

6. System zur Injektion eines Füllstoffs in Fluidphase in eine kanalartige Aufnahme, umfassend:
• eine Patrone (13), die in dichten Fächern (13a, 13b) eine Basis und einen Katalysator enthält, die zu mischen sind, um den Füllstoff in Fluidphase zu erhalten, wobei die Patrone mit einem Mittel (13c) ausgerüstet ist, um die Basis und den Katalysator zu einer Ausgangsöffnung hinauszuführen,
• einen automatischen Mischer (11), der an der Ausgangsöffnung der Patrone (13) angeordnet und dazu konfiguriert ist, die Basis und den Katalysator zu mischen,
• ein Ansatzstück (12) zur intraoralen Injektion, das am oberen Ende des automatischen Mischers (11) angeordnet ist,
**dadurch gekennzeichnet, dass** das Injektionsansatzstück eine Röhre ist, deren distales Ende einen äußeren Durchmesser von kleiner oder gleich 1,5 mm bei einer Länge von größer oder gleich 8 mm hat, dass das Injektionsansatzstück aus einem Formgedächtnismaterial ausgeführt ist, so dass es in einer gewünschten Ausrichtung gebogen werden kann, und dass das Injektionsansatzstück an seiner Außenfläche Folgendes umfasst:
• entweder kreisförmige Rippen (1200), die eine Kehle definieren, wobei das obere Ende des automatischen Mischers (11) so in diese Kehle geformt ist, dass das Injektionsansatzstück in dem oberen Ende in Position arretiert ist, wobei es einen Drehfreiheitsgrad um seine Symmetrieachse (A) beibehält,
• oder eine oder mehrere kreisförmige, zueinander parallele Rippen (1201), wobei das obere Ende des automatischen Mischers (11) so auf diese kreisförmige Rippe geformt ist, dass das Injektionsansatzstück in dem oberen Ende in Position arretiert ist, wobei es einen Drehfreiheitsgrad um seine Symmetrieachse (A) beibehält.

7. System nach Anspruch 6, wobei das Injektionsansatzstück (12) eine zylindrische Röhre ist, deren distales Ende einen externen Durchmesser zwischen 0,75 mm und 0,95 mm hat.

8. System nach einem der Ansprüche 6 oder 7, wobei das Injektionsansatzstück (12) eine zylindrische Röhre ist, deren distales Ende einen externen Durchmesser von 0,8 mm hat.

9. System nach einem der Ansprüche 6 bis 8, wobei das Injektionsansatzstück (12) aus Metall ist.

10. System nach einem der Ansprüche 6 bis 9, wobei das Injektionsansatzstück (12) eine Länge von größer oder gleich 15 mm hat.

11. Einsatzbereites zahnärtzliches Kit, umfassend:
• eine Patrone (13), die in dichten Fächern (13a, 13b) eine Basis und einen Katalysator enthält, die zu mischen sind, um den Füllstoff in Fluidphase zu erhalten, wobei die Patrone mit einem Mittel (13c) ausgerüstet ist, um die Basis und den Katalysator zu einer Ausgangsöffnung hinauszuführen,
• die Injektionsvorrichtung nach Ansprüchen 1 bis 5.

## Claims

1. Device for injecting a fluid-phase filling material into a canal space, the said device comprising an adaptor tip (10), an automixer (11) connected to the said adaptor tip, an intra-oral injection tip (12) arranged at the upper end of the said automixer, **characterized in that** the said injection tip is a tube the distal end of which has an external diameter less than or equal to 1.5 mm over a length greater than or equal to 8 mm,
**in that** the said injection tip is made of a shapememory material so that it can be bent to a desired orientation,
and **in that** the said injection tip on its exterior surface comprises:
• either circular ribs (1200) defining a groove, the upper end of the automixer (11) being moulded into this groove so that the said injection tip is locked in position in the said upper end while at the same time maintaining a degree of freedom to rotate about its axis of symmetry (A),
• one or more mutually parallel circular ribs (1201), the upper end of the automixer (11) being moulded over this circular rib so that the said injection tip is locked in position in the said upper end while maintaining a degree of freedom to rotate about its axis of symmetry (A).

2. Device according to Claim 1, in which the injection tip (12) is a cylindrical tube the distal end of which has an external diameter of between 0.75 mm and 0.95 mm.

3. Device according to either of the preceding claims, in which the injection tip (12) is a cylindrical tube the distal end of which has an external diameter of 0.8 mm.

4. Device according to one of the preceding claims, in which the injection tip (12) is made of metal.

5. Device according to one of the preceding claims, in which the injection tip (12) has a length greater than or equal to 15 mm.

6. System for injecting a fluid-phase filling material into a canal space, comprising:
• a cartridge (13) containing, in sealed compartments (13a, 13b), a base and a catalyst that have to be mixed in order to obtain the said fluid-phase filling material, the said cartridge being equipped with a means (13c) for causing the said base and the said catalyst to exit towards an outlet orifice,
• an automixer (11) arranged at the outlet orifice of the cartridge (13) and configured to mix the base and the catalyst,
• an intra-oral injection tip (12) arranged at the upper end of the automixer (11),
**characterized in that** the said injection tip is a tube the distal end of which has an external diameter less than or equal to 1.5 mm over a length greater than or equal to 8 mm,
**in that** the said injection tip is made of a shapememory material so that it can be bent to a desired orientation,
and **in that** the said injection tip on its exterior surface comprises:
• either circular ribs (1200) defining a groove, the upper end of the automixer (11) being moulded into this groove so that the said injection tip is locked in position in the said upper end while at the same time maintaining a degree of freedom to rotate about its axis of symmetry (A),
• one or more mutually parallel circular ribs (1201), the upper end of the automixer (11) being moulded over this circular rib so that the said injection tip is locked in position in the said upper end while maintaining a degree of freedom to rotate about its axis of symmetry (A).

7. System according to Claim 6, in which the injection tip (12) is a cylindrical tube the distal end of which has an external diameter of between 0.75 mm and 0.95 mm.

8. System according to either of Claims 6 and 7, in which the injection tip (12) is a cylindrical tube the distal end of which has an external diameter of 0.8 mm.

9. System according to one of Claims 6 to 8, in which the injection tip (12) is made of metal.

10. System according to one of Claims 6 to 9, in which the injection tip (12) has a length greater than or equal to 15 mm.

11. Ready-to-use dental kit comprising:
• a cartridge (13) containing, in sealed compartments (13a, 13b), a base and a catalyst that have to be mixed in order to obtain a fluidphase filling material, the said cartridge being equipped with a means (13c) for causing the said base and the said catalyst to exit towards an outlet orifice,
• the injection device according to Claims 1 to 5.
